# EUROPEAN PATENT SPECIFICATION

(11) **EP 3 940 052 B1**
(45) Date of publication and mention of the grant of the patent: **13.05.2026**
(21) Application number: 19919371.5
(22) Date of filing: 19.11.2019
(51) Int. Cl.: C12M 1/00, C07H 21/00, C12M 1/36, C12M 1/34, C12N 15/00, G01N 35/10

(54) **NUCLEIC ACID EXTRACTION APPARATUS**
VORRICHTUNG ZUR NUKLEINSÄUREEXTRAKTION
APPAREIL D'EXTRACTION D'ACIDE NUCLÉIQUE

(30) Priority: 12.03.2019 CN 201910184157; 12.03.2019 CN 201920311623 U
(43) Date of publication of application: 19.01.2022
(73) Proprietor: Chen, Hui, Zhejiang 315016 (CN)
(72) Inventor: Chen, Hui, Zhejiang 315016 (CN)
(74) Representative: Chung, Hoi Kan
(86) International application number: PCT/CN2019/119366
(87) International publication number: WO 2020/181825

(56) References cited:
- EP-A1- 1 563 886
- EP-A1- 1 693 454
- EP-A1- 2 813 579
- WO-A1-2014/026008
- CN-A- 101 143 884
- CN-A- 103 305 412
- CN-A- 105 296 327
- CN-A- 108 949 505
- CN-A- 109 142 708
- CN-Y- 201 176 432
- JP-A- 2006 197 850
- JP-U- 3 220 438

## Description

### Field of the Invention

The invention relates to the technical field of biotechnologies, in particular to a type of nucleic acid extraction device.

### Background of the Invention

Nucleic acid extraction is an important technology in life science research, biotechnology application and genetic diagnosis. Early nucleic acid extraction is established on the principle of phenol chloroform extraction for impurity removal and precipitation of nucleic acid with ethanol, but this method is very complex.

At present, commercial nucleic acid extraction methods are mainly established on a solid phase adsorption method, in other words, nucleic acid molecules are adsorbed onto the surface of a certain solid phase adsorbent under the specific condition (e.g. pH and specific salt), and then elution purification methods are adopted, wherein two methods are mainly included: (1) Spin-column method: Spin-columns are filled with silicon-containing materials or other solids capable of adsorbing nucleic acid serving as media, guanidine salt and other chaotropic salt serve as binding agents; when passing through the spin-columns, the nucleic acid molecules are adsorbed onto the solid phases, then impurities are washed away through a solution containing ethanol, and finally nucleic acid is eluted out with a low-salt solution; and the method is simple and convenient, high in extraction efficiency and good in extraction effect, is a nucleic acid purification method most commonly used at present, but has the defects that repeated centrifugation is needed in the extraction process, and high-throughput and automatic operation is not convenient. (2) Magnetic bead method: Magnetic microspheres with surfaces wrapped with silicon materials or other materials capable of being bound with nucleic acid are used as solid phase carriers to separate DNA and RNA from samples under the action of chaotropic salt (guanidinium chloride, guanidinium isothiocyanate and the like) and external application of magnetic fields; and the method easily achieves automatic and high-throughput operation, but has the defects that the cost is high, time is wasted in the extraction process, thus, time consumed for whole genetic detection is too long, and doctors and patients cannot obtain detection results in time. The chemical stability of magnetic beads is poor. The extraction efficiency is easily affected, and the change of magnetic bead extraction effects of different samples, different manufacturers and different batches is large. As a result, extraction and purification results are not stable. In addition, cross-contamination is easily caused during repeated cleaning of the magnetic bead in the magnetic bead extraction process, leading to false positive results. Existing nucleic acid automatic extraction apparatuses are usually based on the magnetic bead method, such as nucleic acid extraction apparatuses disclosed in the China invention patent with the patent number of ZL2016020294747.7 (published application number of CN205635634U) and the China invention patent with the patent number of ZL201610687392.2 (published application number of CN106119082A). Japanese utility model JP3220438U discloses a sample extraction device having an extraction workbench and a movable operation module, wherein the adsorption process of nucleic acids on the adsorbent medium and the washing process of impurities on the adsorption membrane are accomplished by the movement of a syringe piston to draw in and press out liquid from a regular combination column.

### Content of the Invention

The first technical problem to be solved by the invention is to provide a nucleic acid extraction device simple in structure and convenient to use for the prior art.

The second technical problem to be solved by the invention is to provide a nucleic acid extraction device capable of extracting nucleic acid of high purity for the prior art.

The third technical problem to be solved by the invention is to provide a nucleic 20 acid extraction device free of cross-contamination among samples in the nucleic acid extraction process for the prior art.

The technical solution adopted by the invention to solve the above technical problem is: A type of nucleic acid extraction device comprises a workbench with a tabletop, and is characterized by further including:
a sliding seat arranged on the tabletop and capable of being driven by a sliding assembly to slide along the tabletop back and forth;
a sample support arranged on the sliding seat and provided with a plurality of sample holes formed side by side in a direction perpendicular to a sliding direction of the sliding seat, wherein the sample holes are used for containing sample tubes;
a reagent strip support arranged on the sliding seat and provided with containing grooves for containing reagent strips, wherein the number of the containing grooves corresponds to that of the sample holes,
each reagent strip comprising the first sucker holes, the second sucker holes and reagent holes which are arranged linearly; wherein, the first sucker holes are used for containing sucker assemblies, the second sucker holes are used for containing suckers, the reagent holes comprise the first reagent holes for containing diluent, the second reagent holes for containing cleaning fluid, the third reagent holes for containing eluant and the fourth reagent holes for storing target substances, the number of the second reagent holes is at least two, and the first sucker holes, the second sucker holes and the reagent holes in the reagent strips are opposite to the corresponding sample holes correspondingly when the reagent strips are put in the containing grooves;
a liquid transfer table horizontally arranged on a vertical frame fixed to the tabletop and capable of being driven by a lifting assembly to go up and down relative to the sliding seat;
piston assemblies arranged on the liquid transfer table, wherein the piston assemblies comprise piston parts and piston driving apparatuses, the number of the piston parts corresponds to that of the sample holes, the piston parts comprise piston tubes and pistons arranged in the piston tubes and capable of moving along inner walls of the piston tubes up and down, the piston tubes all vertically penetrate through the liquid transfer table, the first connectors for being in inserted connection with the suckers are formed at lower ends of the piston tubes correspondingly, and the piston driving apparatuses can drive the pistons in the piston parts to move relative to the corresponding piston tubes up and down; and
suction assemblies comprising connecting suction tubes and vacuum pumps, wherein the number of the connecting suction tubes corresponds to that of the sample holes, the connecting suction tubes all penetrate through the liquid transfer table, upper ends of the connecting suction tubes connects with the vacuum pumps correspondingly, wherein the sucker assemblies comprise filter element suckers and disposable capillary suction tubes, adsorption filter elements are embedded into the filter element suckers, upper ends of the disposable capillary suction tubes sleeve lower ends of the connecting suction tubes and are embedded into upper ports of the filter element suckers, and the disposable capillary suction tubes are located in the filter element suckers and located above the absorption filter elements.

In order to conveniently conduct heating and heat preservation on samples according to operation requirements, a heating device for heating the sample support is arranged on the sliding seat.

In order to conveniently puncture seals on the reagent strips, a puncture assembly for puncturing the seals on the reagent holes is further comprised, the puncture assembly comprises puncture frames and puncture heads installed on the puncture frames, the puncture heads are matched with the sample holes, the pistons are all fixed to piston frames, the piston frames are fixed to output shafts of the piston driving apparatuses, and the puncture frames are fixed to the piston frames. Thus, when the piston driving apparatuses drive the piston frames to move downwards, the pistons are driven to move downwards, meanwhile the puncture frames are driven to move downwards, and then the puncture heads can move downwards to puncture the seals on the reagent strips.

In order to better perform suction actions, the plurality of vacuum pumps correspond to the piston parts one to one, suction connectors are arranged on the piston frames, the suction connectors correspond to the piston parts one to one in number, the suction connectors all penetrate through the piston frames, one ends of suction connectors are connected with connectors on the corresponding vacuum pumps through connecting tubes, and the other ends of the suction connectors are connected with upper-end connectors of the connecting suction tubes through connecting tubes.

In order to enable the vacuum pumps to be more stably installed, an installation frame is arranged on the tabletop, and the vacuum pumps are put on the installation frame respectively.

In order to better enable the sliding seat to be driven by the sliding assembly along the tabletop, the sliding assembly comprises the first sliding blocks, the first sliding rails, a pressing block, a sliding motor, a synchronous belt, a driving belt wheel and a driven belt wheel, the sliding blocks are fixed to the bottom of the sliding seat, the first sliding rails are fixed to the tabletop, the first sliding blocks can slide along the first sliding rails back and forth, the sliding motor is fixed to the tabletop, the driving belt wheel is fixed to an output shaft of the sliding motor, the driven belt wheel is installed on the tabletop, the synchronous belt is tensioned between the driving belt wheel and the driven belt wheel, and the pressing block is fixed to one side of the sliding seat and pressed on the synchronous belt.

Preferably, the reagent strips comprise carrier plates, a plurality of the first through holes and the second through holes are formed in the carrier plates in a length direction of the carrier plates side by side, tube bodies corresponding to the first through holes one to one stretch out of one sides of the carrier plates; meanwhile, sleeve bodies corresponding to the second through holes one to one further stretch out of one sides of the carrier plates, tube openings of the tube bodies are right opposite to the corresponding first through holes correspondingly, openings of the sleeve bodies are right opposite to the corresponding second
through holes correspondingly, and therefore the first sucker holes, the second sucker holes and the reagent holes are formed correspondingly.

Furthermore, the first connecting parts and second connecting parts are arranged on two sides of the carrier plates correspondingly, and the first connecting parts and the second connecting parts can be detachably connected.

Furthermore, the first connecting parts are connecting columns, and the second connecting parts are connecting sleeves allowing the connecting columns to be inserted therein.

Preferably, the sucker assemblies comprise filter element suckers and disposable capillary suction tubes, adsorption filter elements are embedded into lower ends of the filter element suckers, upper ends of the disposable capillary suction tubes are wrapped in the lower ends of the connecting suction tubes and are embedded into upper ports of the filter element suckers, and the disposable capillary suction tubes are located in the filter element suckers and located above the absorption filter elements. Due to the arrangement of the disposable capillary suction tubes, the connecting suction tubes and the filter element suckers are separated through the non-contaminated disposable capillary suction tubes, the connecting suction tubes are prevented from coming into contact with the filter element suckers in the suction process, so that the filter element suckers will not be polluted and then the purity of nucleic acid extraction can be guaranteed. In addition, the connecting suction tubes can be repeatedly used due to the arrangement of the disposable capillary suction tubes, and therefore the nucleic acid extraction cost is reduced. In addition, the solution can be rapidly and completely pumped out of the filter elements by the disposable capillary suction tube through the capillary tube effect, and therefore nucleic acid adsorbed on the filter elements and liquid are completely separated and impurities on the filter elements are completely washed away.

In order to better achieve suction, lower ends of the disposable capillary suction tubes are opposite to the adsorption filter elements up and down.

In order to improve the suction effect, distances between the lower ends of the disposable capillary suction tubes and upper surfaces of the adsorption filter elements are 0-10 mm.

Preferably, distances between the lower ends of the disposable capillary suction tubes and the upper surfaces of the adsorption filter elements are 2-5mm, and therefore sufficient suction can be achieved.

Preferably, the disposable capillary suction tubes comprise the first portions located at the upper ends and the second portions located at the lower ends, wherein the first portions are used for being connected to the connecting suction tubes in a sleeving mode, and tube diameters of the second portions enable the liquid to smoothly pass through the disposable capillary suction tubes under the action of suction force. If the tube diameters of the second portions are too large, it is difficult to completely drain the liquid as no capillary effect exists. Besides, part of the liquid may be left between the second portions and the filter element suction tubes. If the tube diameters of the second portions are too small, the liquid may be intercepted in the second portions, so that smooth suction cannot be achieved.

Furthermore, preferably, the tube diameters of the second portions are 0.5-2.0mm.

Preferably, the edges of upper ports of the disposable capillary suction tubes are lower than those of the upper ports of the filter element suckers. By means of above design, two-time sucker withdrawal can be achieved, the filter element suckers and the disposable capillary suction tubes are separated through the first-time sucker withdrawal, the disposable capillary suction tubes can be separated from the connecting suction tubes through the second-time sucker withdrawal. Thus, the filter element suckers can sleeve other suckers to clean the adsorption filter elements in the filter element suckers after sucker withdrawal, and therefore elution of nucleic acid substances is achieved.

In order to facilitate separation between the disposable capillary suction tubes and the filter element suckers, convex rings are arranged on inner circumferential surfaces of upper ends of the filter element suckers in a circumferential direction, and the convex rings abut against outer circumferential surfaces of corresponding positions of the disposable capillary suction tubes. Compared with surface-to-surface contact between inner circumferential surfaces of the disposable capillary suction tubes and outer circumferential surfaces of the connecting suction tubes, friction force of line-to-surface contact between the convex rings and the outer circumferential surfaces of the disposable capillary suction tubes is smaller. By means of the design, the filter element suckers and the disposable capillary suction tubes can withdraw from the connecting suction tubes correspondingly. First the filter element suckers with small friction force withdraw, then the disposable capillary suction tubes withdraw. Due to the arrangement of the convex rings, the disposable capillary suction tubes and the filter element suckers can be sealed. In addition, the adsorption filter elements can be prevented from falling from the filter element suckers through the convex rings.

In order to enable the disposable capillary suction tubes and the filter element suckers to be better sealed, the number of the convex rings arranged up and down is at least two.

In order to further promote sealing between the disposable capillary suction tubes and the filter element suckers, all the convex rings are arranged vertically at intervals.

Compared with the prior art, the invention has the following advantages: The nucleic acid extraction device in the invention comprises the sliding seat and the liquid transfer table, the sample support and the reagent strip support are arranged on the sliding seat, the piston assemblies and the suction assemblies are arranged on the liquid transfer table, sample holes are formed in the sample support, containing grooves are formed in the reagent strip support, the containing grooves correspond to the sample holes one to one in number; when reagent strips are contained in the containing grooves correspondingly, the first sucker holes, the second suction holes and reagent holes in the reagent strips are opposite to the corresponding sample holes correspondingly. Thus, operation of loading of the sucker assemblies and suckers and suction, injection, uniform mixing and the like of samples and reagents may be achieved through up-and-down movement of the liquid transfer table relative to the sliding seat or transverse movement of the sliding seat relative to the liquid transfer table, so as to serve as the basis for the automatic operation of nucleic acid extraction. In addition, due to the arrangement of the disposable capillary suction tubes in the invention, the connecting suction tubes will not come into contact with lower ends of the connecting suction tubes so that the connecting suction tubes will be prevented from being contaminated in the suction process. Thus, contamination among different nucleic acid extraction samples can be avoided, and the connecting suction tubes can be repeatedly used.

### Description of Attached Figures

Figure 1 is a structural schematic diagram of a nucleic acid extraction device in an embodiment of the invention;
Figure 2 is a structural schematic diagram of another direction of Figure 1;
Figure 3 is a local structural schematic diagram of a nucleic acid extraction device in an embodiment of the invention;
Figure 4 is an enlarged view of part I in Figure 3;
Figure 5 is a structural schematic diagram of another direction of Figure 3;
Figure 6 is a structural schematic diagram of another direction of Figure 3;
Figure 7 is another local structural schematic diagram of a nucleic acid extraction device in an embodiment of the invention;
Figure 8 is another local structural schematic diagram of a nucleic acid extraction device in an embodiment of the invention;
Figure 9 is a structural schematic diagram of a sucker withdrawal plate in an embodiment of the invention;
Figure 10 is a structural schematic diagram of a reagent strip support in an embodiment of the invention;
Figure 11 is a structural schematic diagram of a sample support in an embodiment of the invention;
Figure 12 is the internal structure of a piston part in an embodiment of the invention;
Figure 13 is a structural schematic diagram of a reagent strip in an embodiment of the invention;
Figure 14 is a structural schematic diagram of another direction of Figure 13;
Figure 15 is a structural schematic diagram of another direction of Figure 13;
Figure 16 is a structural schematic diagram of a sucker assembly in an embodiment of the invention;
Figure 17 is a cutaway view of a sucker assembly in an embodiment of the invention;
Figure 18 is an enlarged view of part II in Figure 17;
Figure 19 is a structural schematic diagram of a filter element sucker in an embodiment of the invention;
Figure 20 is the internal structure of a sucker assembly in an embodiment of the invention.

### Specific Implementation Modalities

The embodiments in conjunction with the attached figures further describe the invention.

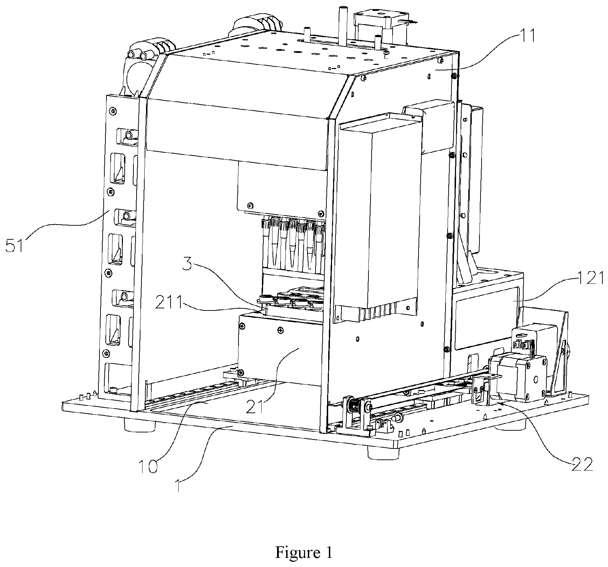

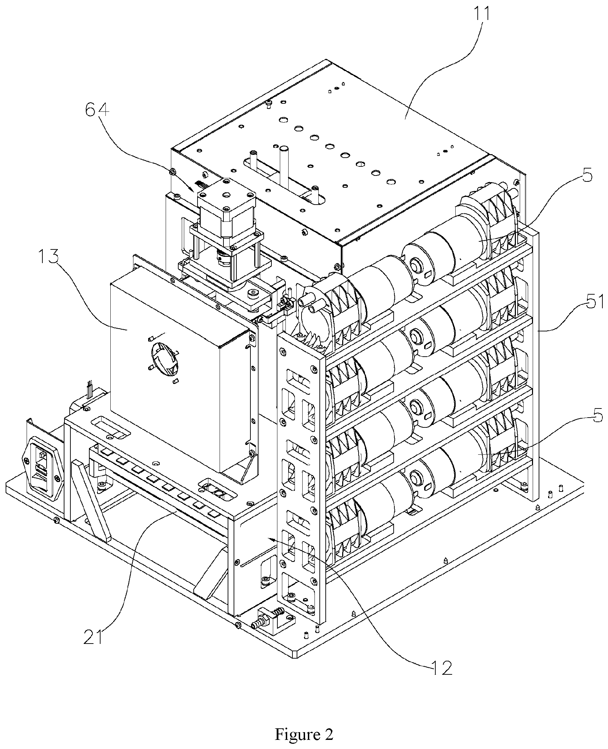

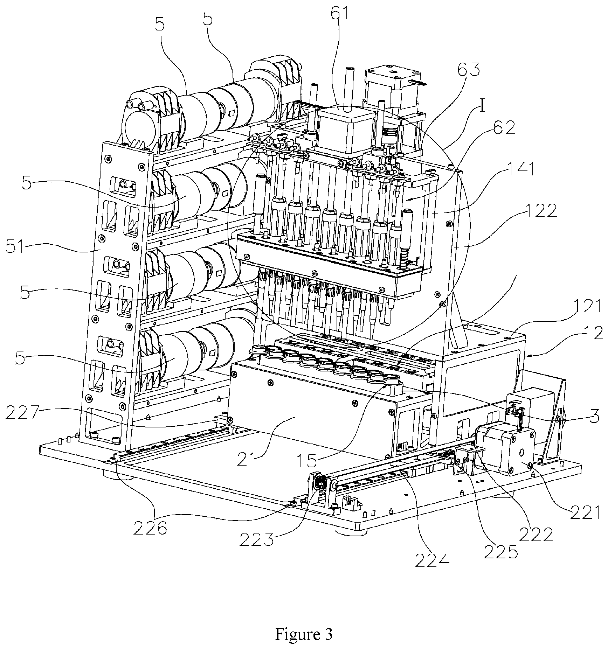

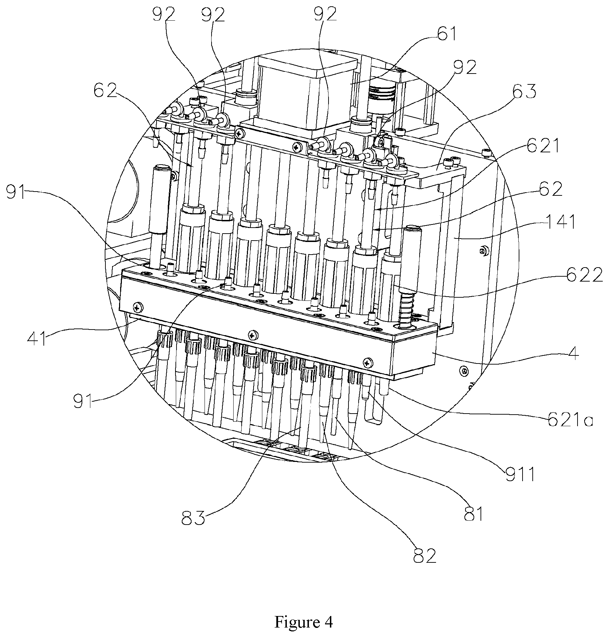

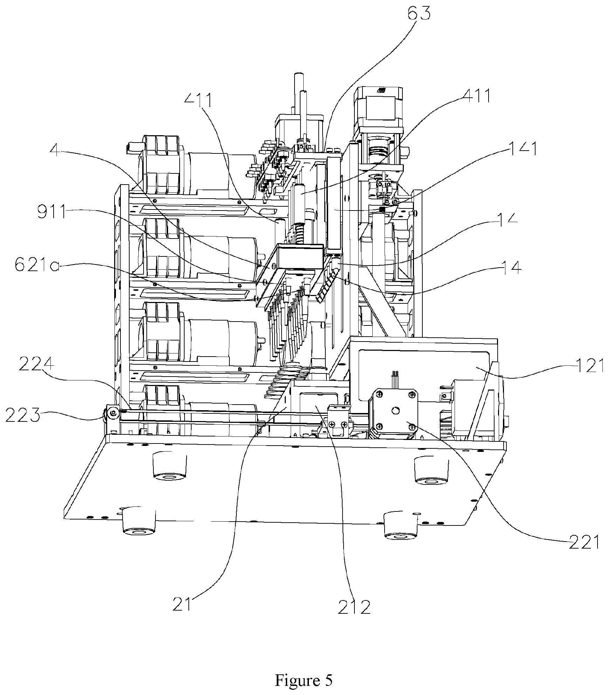

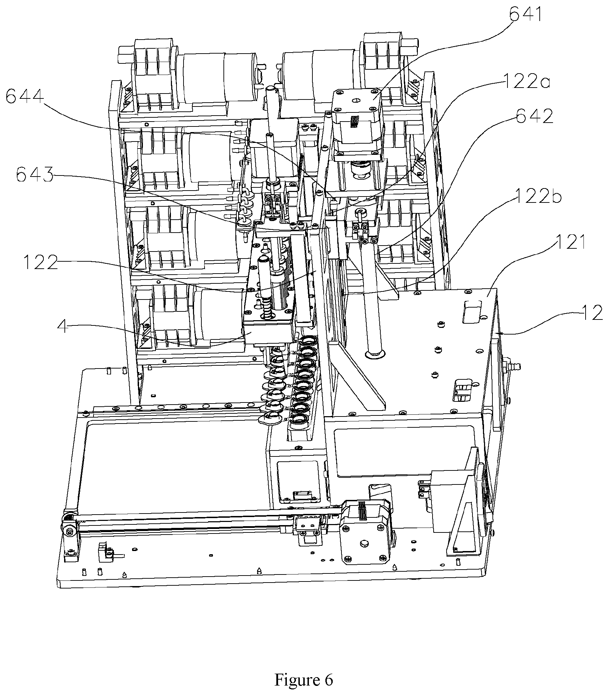

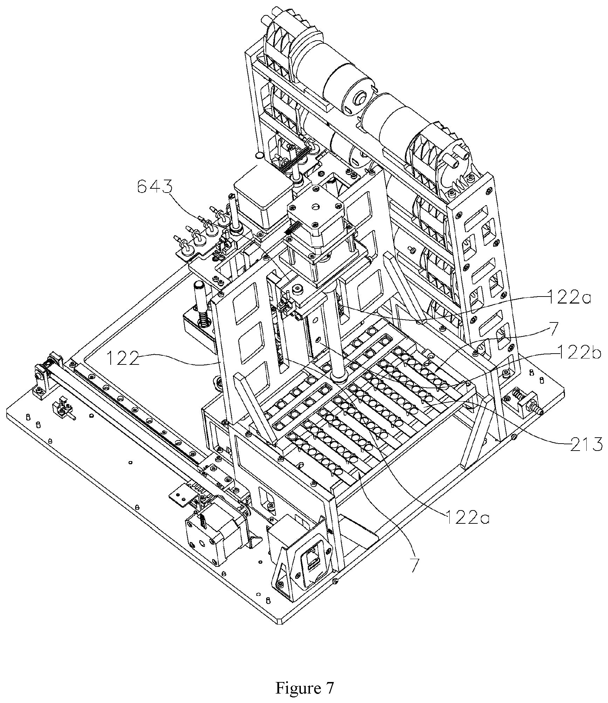

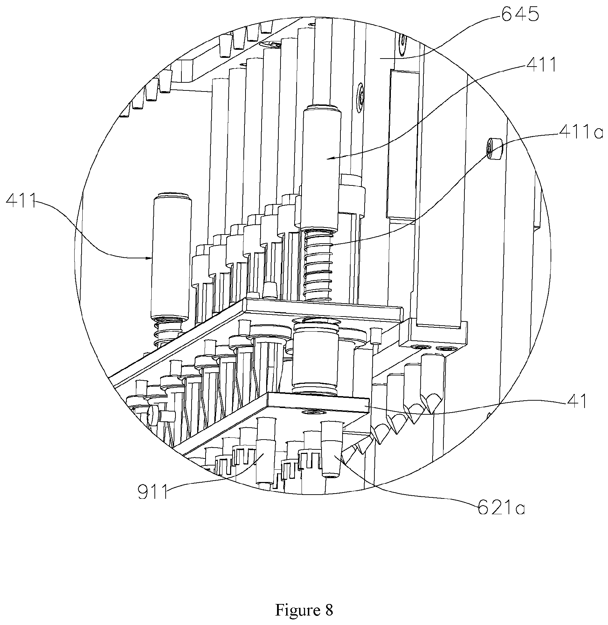

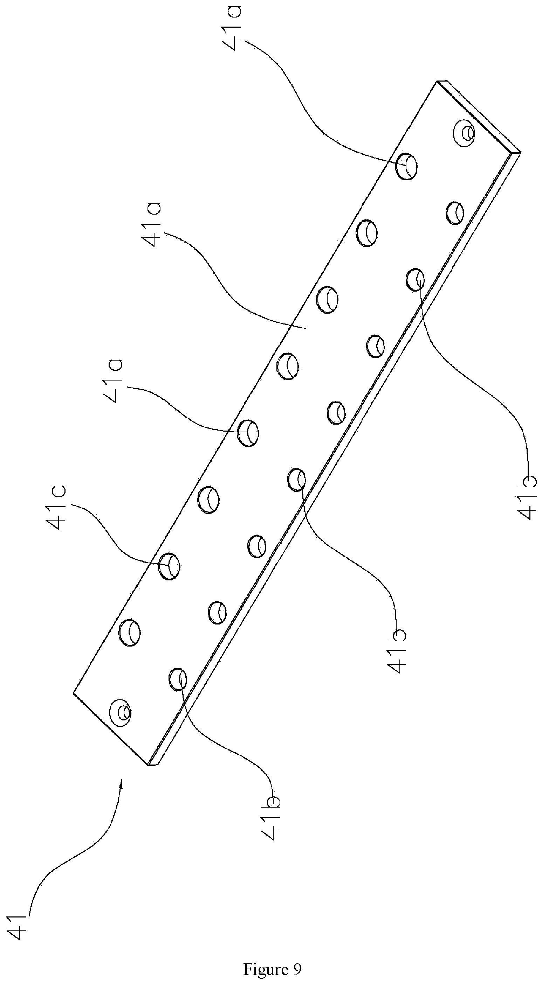

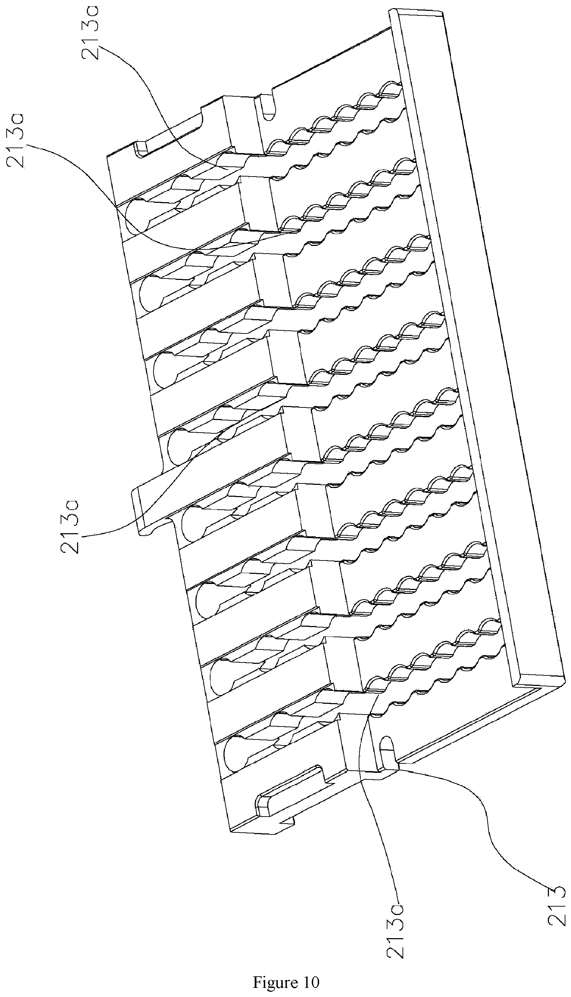

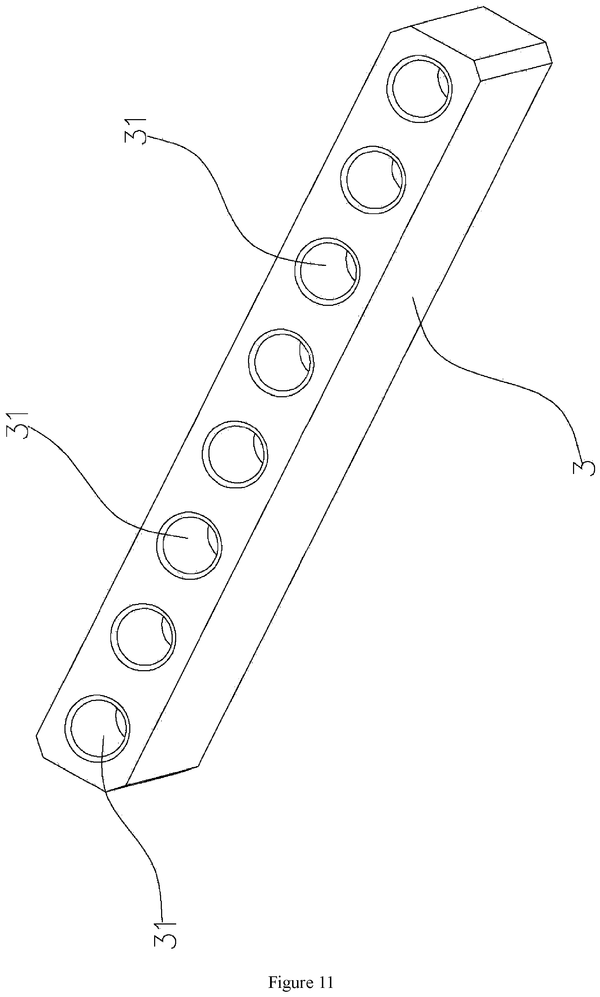

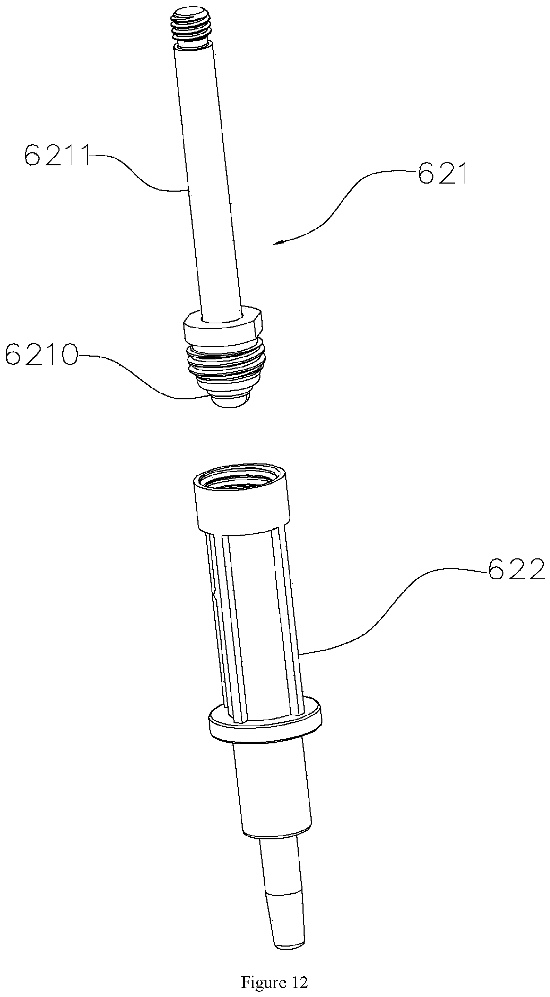

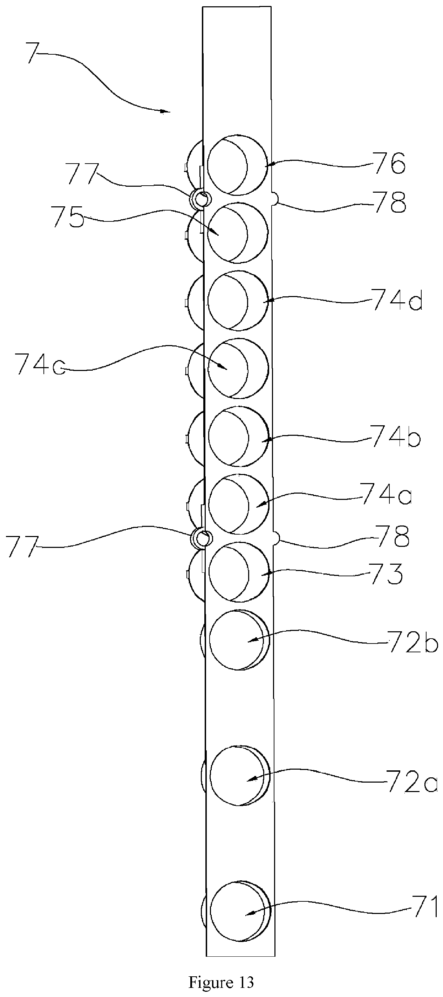

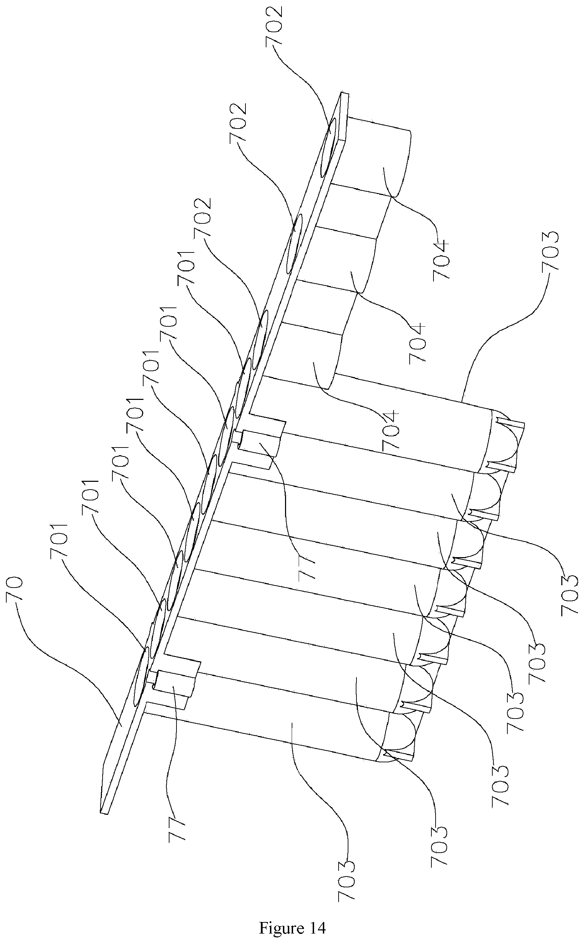

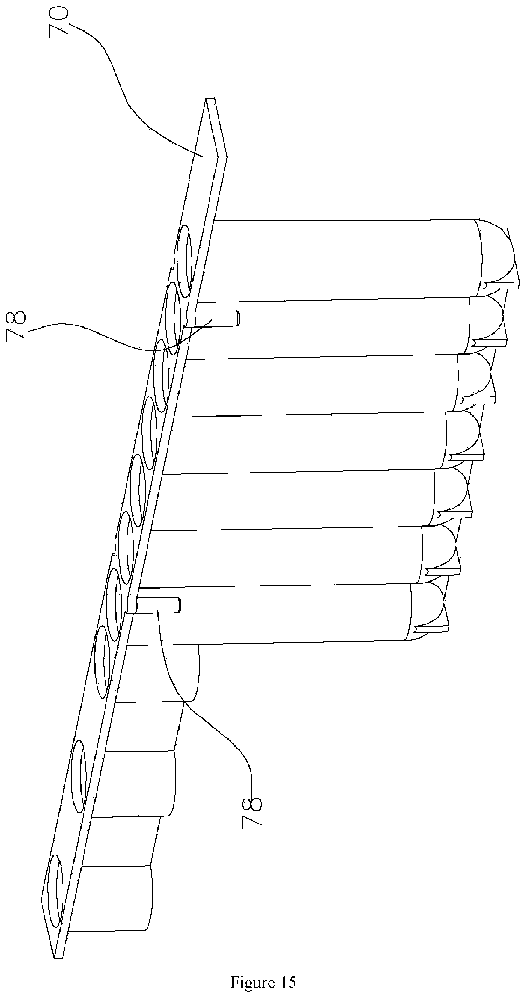

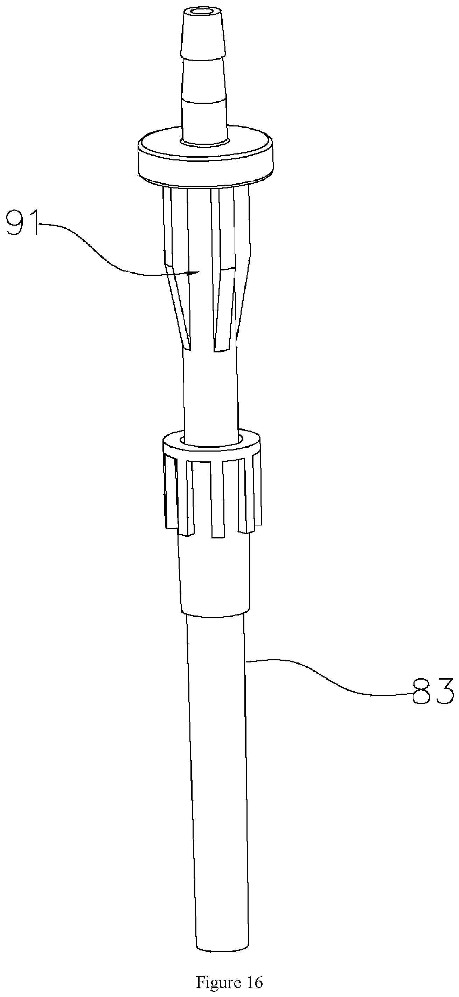

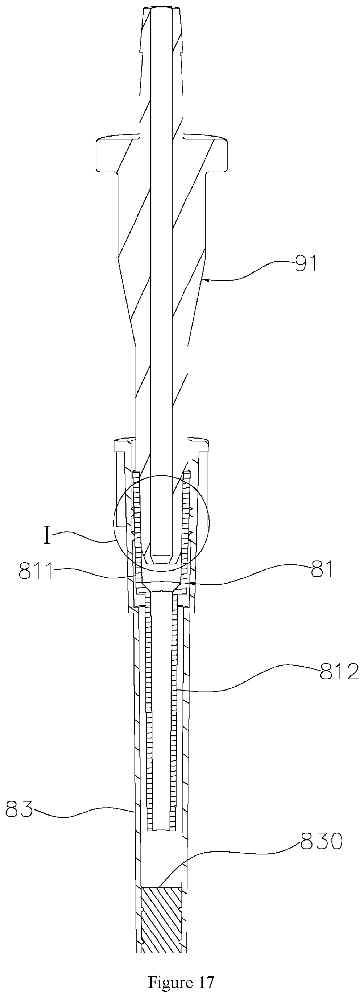

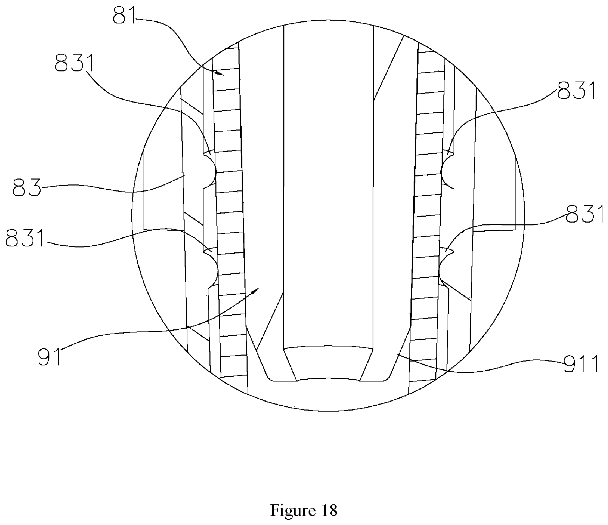

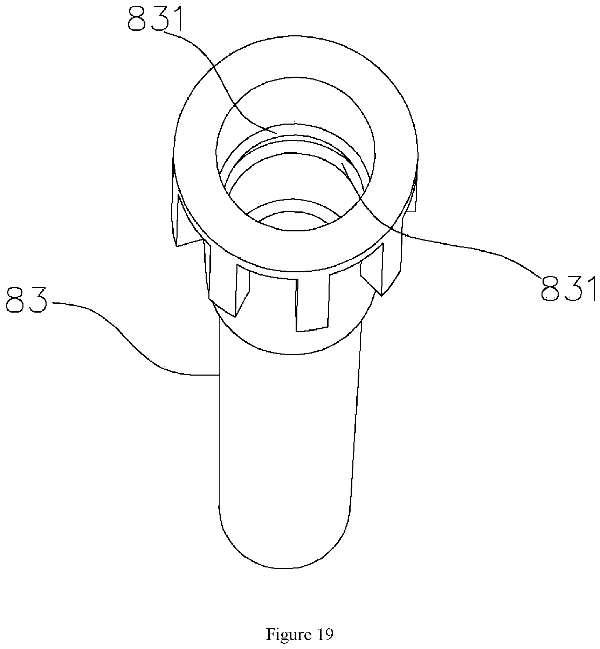

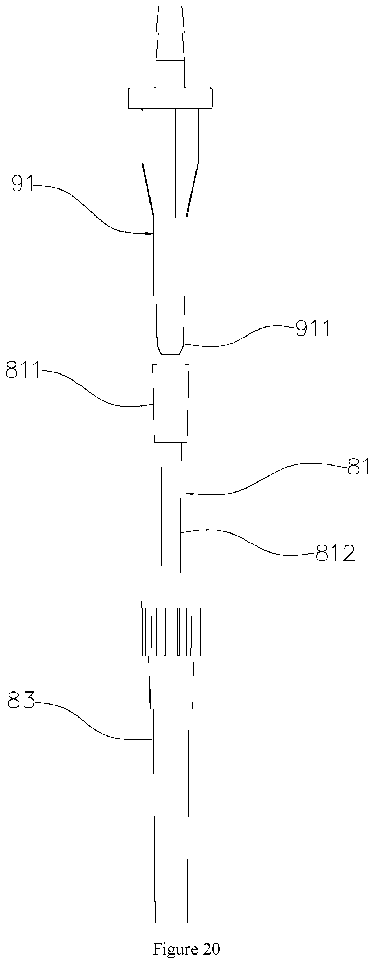

As shown in Figures 1-20, a type of nucleic acid extraction device comprises a workbench 1 with a tabletop 10, a sliding seat 21, a sample support 3, a reagent strip support 213, a liquid transfer table 4, piston assemblies and suction assemblies. In the embodiment, a shell 11 is arranged on the tabletop 10, the sliding seat 21 and the liquid transfer table 4 are both arranged in the shell 11, and in addition, a cooling fan 13 is arranged on the back of the shell 11.

The sliding seat 21 is arranged on the tabletop 10, and the sliding seat can be driven by a sliding assembly 22 to slide along the tabletop 10 back and forth. In the embodiment, in order to better enable the sliding seat 21 to be driven by the sliding assembly 22 to slide along the tabletop 10, the sliding assembly 22 comprises the first sliding blocks 227, the first sliding rails 226, a pressing block 225, a sliding motor 221, a synchronous belt 224, a driving belt wheel 222 and a driven belt wheel 223; the sliding blocks 227 are fixed to the bottom of the sliding seat 21, the first sliding rails 226 are fixed to the tabletop 10, the first sliding blocks 227 can slide along the first sliding rails 226 back and forth, the sliding motor 221 is fixed to the tabletop 10, the driving belt wheel 222 is fixed to an output shaft of the sliding motor 221, the driven belt wheel 223 is installed on the tabletop 10, the synchronous belt 224 is tensioned between the driving belt wheel 222 and the driven belt wheel 223, and the pressing block 225 is fixed to one side of the sliding seat 21 and pressed on the synchronous belt 224. In the embodiment, the sliding motor 221 is a stepping motor.

The sample support 3 is arranged on the sliding seat 21, the sample support 3 is provided with a plurality of sample holes 31 formed side by side in a direction perpendicular to a sliding direction of the sliding seat 21, and the sample holes 31 are used for containing sample tubes 15. In the embodiment, the sample support 3 is in a long block shape, the sample holes 31 are evenly distributed in the sample support in a length direction at intervals, and the sample holes 31 stretch in a height direction of the sample support 3 to form blind holes. In order to facilitate heating and heat preservation on samples according to operation requirements, a heating device for heating the sample tubes 15 put in the sample holes 31 is arranged on the sliding seat 21. Specifically, in the embodiment, the heating device is a heating pool 211 arranged at the front end of the sliding seat 21, the sample support 3 is contained in the heating pool 211, and therefore the samples on the sample support 3 can be sufficiently subjected to heating and heat preservation.

The reagent strip support 213 is arranged at the rear end of the sliding seat 21, containing grooves 213a for containing reagent strips 7 are formed in the reagent strip support, the containing grooves 213a of the reagent strips 7 correspond to the sample holes 31 one to one in number, each reagent strip 7 comprises the first sucker hole 71, the second sucker holes 72a and 72 b and a reagent hole which are formed linearly, the first sucker holes 71 are used for containing sucker assemblies, the second sucker holes 72a and 72b are used for containing suckers 82, the reagent holes comprise the first reagent holes 73 for containing diluent, the second reagent holes 74a, 74b, 74c and 74d for containing cleaning fluid, the third reagent holes 75 for containing eluant and the fourth reagent holes 76 for storing target substances, and the first sucker holes 71, the second sucker holes 72a and 72b and the reagent holes in the reagent strips 7 are opposite to the corresponding sample holes 31 correspondingly when the reagent strips 7 are put in the containing grooves 213a. In the embodiment, the reagent strips 7 comprise carrier plates 70, a plurality of the first through holes 701 and the second through holes 702 are formed in the carrier plates 70 in a length direction of the carrier plates side by side, tube bodies 703 corresponding to the first through holes 701 one to one stretch out of one sides of the carrier plates 70; meanwhile, sleeve bodies 704 corresponding to the second through holes 702 one to one further stretch out of one sides of the carrier plates 70, tube openings of the tube bodies 703 are right opposite to the corresponding first through holes 701 correspondingly, openings of the sleeve bodies 704 are right opposite to the corresponding second through holes 702 correspondingly, and therefore the first sucker holes 71, the second sucker holes 72a and 72b and the reagent holes are formed correspondingly. Furthermore, the cleaning fluid, namely the first cleaning fluid, the second cleaning fluid, the third cleaning fluid and the fourth cleaning fluid are contained in the second reagent holes 74a, 74b, 74c and 74d so that the quality of nucleic acid extraction can be effectively guaranteed through a plurality of times of rinsing in the nucleic acid extraction process.

Furthermore, the first connecting parts 78 and second connecting parts 77 are arranged on two sides of the carrier plates 70 correspondingly, and the first connecting parts 78 and the second connecting parts 77 can be detachably connected. Thus, the two or more reagent strips 7 can be connected through the first connecting parts 78 and the second connecting parts 77, the connected and combined reagent strips 7 can be vertically put in an upright mode, it is not necessary to arrange other devices to contain the reagent strips 7, and therefore use of the reagent strips 7 is facilitated. The first connecting parts 78 and the second connecting parts 77 can be detachably connected through a plurality of specific implementation modes, preferably, the first connecting parts 78 are connecting columns, and the second connecting parts 77 are connecting sleeves allowing the connecting columns to be inserted therein. Specifically, the first connecting parts 78 are fixed to bottoms of side edges of one sides of the carrier plates 70, the first connecting parts 78 stretch in a vertically-downward mode to be suspended, the second connecting parts 77 are fixed to bottoms of side edges of the other sides of the carrier plates 70 and/or corresponding tube walls, on the sides, of the corresponding tube bodies 703, the second connecting arts 77 stretch in a vertically-downward mode, and openings of the second connecting parts are exposed outside.

The liquid transfer table 4 is horizontally arranged on a vertical frame 12 of the tabletop 10, and can be driven by a lifting assembly 64 to be lifted and lowered relative to the sliding seat 21. In order to better enable the liquid transfer table 4 to be driven by the lifting assembly 64 to go up and down, the lifting assembly 64 comprises a lifting motor 641, a lifting lead screw 642 and a lifting seat 643, the vertical frame 12 comprises an n-shaped frame 121 and a vertical plate 122 fixed to a top plate of the n-shaped frame 121, the lifting motor 641 is installed at the upper end of the vertical plate 122, the second sliding rail 122b and a sliding hole 122a vertically stretch from the position, below the lifting motor 641, of the vertical plate 122, the lifting lead screw 642 is vertically arranged, the upper end of the lifting lead screw is connected with an output shaft of the lifting motor 641, the lower end of the lifting lead screw is connected with the top plate of the n-shaped frame 121, the lifting seat 643 penetrates through the vertical plate 122 through the sliding hole 122a, the front end of the lifting seat is fixed to the liquid transfer table 4, the rear end of the lifting seat sleeves the lead screw 642, a screw transmission pair is formed by the lifting seat and the lead screw 642, the second sliding block 644 is arranged on the lifting seat 643, and when the lifting motor 641 drives the lead screw 642 to rotate, the lead screw 642 can drive the lifting seat 643, and enables the second sliding block 644 to slide along the second sliding rail 122b up and down, and the corresponding position of the lifting seat 643 can move along the sliding hole 122a up and down. In an initial state, the reagent strip support 231 is located below the n-shaped frame 121.

The piston assemblies are arranged on the liquid transfer table 4 and comprise piston parts 62 and piston driving apparatuses 61. In the embodiment, the piston driving apparatuses 61 are motors, the number of the piston parts 62 is consistent with that of the sample holes 31, the piston parts 62 comprise piston tubes 622 and pistons 621 arranged in the piston tubes 622 and capable of moving along inner walls of the piston tubes 622 up and down, the piston tubes 622 all vertically penetrate through the liquid transfer table 4, the first connectors 621a for being in inserted connection with the suckers 82 are formed at lower ends of the piston tubes 622 correspondingly, and the piston driving apparatuses 61 can drive the pistons 621 in the piston parts 62 to move relative to the corresponding piston tubes 622 up and down. In the embodiment, the piston driving apparatuses 61 are penetration type stepping motors, the pistons 621 are all fixed to piston frames 63, the pistons 621 comprise piston bodies 6210 and piston rods 6211 fixed to top ends of the piston bodies 6210, and the piston frames 63 are fixed to output shafts of the piston driving apparatuses 61. In order to enable all vacuum pumps 5 to be stably installed, an installation frame 51 is arranged on the tabletop 10, and the vacuum pumps 5 are put on the installation frame 51.

The suction assemblies comprise connecting suction tubes 91 and vacuum pumps 5, wherein the number of the connecting suction tubes 91 is consistent with that of the sample holes 31, the connecting suction tubes 91 all penetrate through the liquid transfer table 4, upper ends of the connecting suction tubes 91 connect with the vacuum pumps 5 correspondingly, and the second connectors 911 for being in inserted connection with the sucker assemblies are formed at lower ends of the connecting suction tubes correspondingly. In order to better achieve suction actions, the plurality of vacuum pumps 5 correspond to the connecting suction tubes 91 one to one, suction connectors 92 are arranged on the piston frames 63, the suction connectors 92 correspond to the connecting suction tubes 91 one to one in number, the suction connectors 92 all penetrate through suction connector frames 65, one ends of the suction connectors 92 are connected with connectors on the corresponding vacuum pumps 5 through connecting tubes (not shown), and the other ends of the suction connectors are connected with upper-end connectors of the connecting suction tubes 91 through connecting tubes (not shown).

The sucker assemblies comprise filter element suckers 83 and disposable capillary suction tubes 81, wherein lower ports of the connecting suction tubes 91 are inserted into upper ends of the filter element suckers 83, and adsorption filter elements 830 are embedded into lower ends of the filter element suckers 83. Upper ends of the disposable capillary suction tubes 81 sleeve lower ends of the connecting suction tubes 91 and are embedded into upper ports of the filter element suckers 83, the disposable capillary suction tubes 81 are located in the filter element suckers 83 and located above the absorption filter elements 830, and lower ends of the disposable capillary suction tubes 81 are opposite to the adsorption filter elements 830 up and down. Thus, in the embodiment, due to the arrangement of the disposable capillary suction tubes 81, the situation that the connecting suction tubes 91 come into contact with the filter element suckers 83 can be avoided so that the filter element suckers 83 can be prevented from being contaminated, and then the purity of nucleic acid extraction can be guaranteed.

Furthermore, the lower ends of the disposable capillary suction tubes 81 are opposite to the adsorption filter elements 830 up and down, and in order to improve the suction effect to the maximum degree, the lower ends of the disposable capillary suction tubes 81 are kept spaced from upper surfaces of the adsorption filter elements 830 by a proper distance. In the embodiment, in order to achieve sufficient suction, distances between the lower ends of the disposable capillary suction tubes 81 and the upper surfaces of the adsorption filter elements 830 are 4mm.

The disposable capillary suction tubes 81 comprise the first portions 811 located at the upper ends and the second portions 812 located at the lower ends, wherein the first portions 811 are used for being connected to the connecting suction tubes 91 in a sleeving mode, and tube diameters of the second portions 812 enable the liquid to smoothly pass through the disposable capillary suction tubes 81 under the action of suction force. If the tube diameters of the second portions 812 are too large, the capillary tube effect of the second portions 812 is not remarkable, the liquid may be left between the second portions 812 and the connecting suction tubes 91, or otherwise, if the tube diameters of the second portions 812 are too small, the liquid may be intercepted in the second portions 812, and smooth suction cannot be achieved. In the embodiment, preferably, the tube diameters of tube bodies of the second portions 812 are 0.5-2.0mm.

Furthermore, in order to achieve two-time sucker withdrawal, edges of upper ports of the disposable capillary suction tubes 81 are lower than those of upper ports of the filter element suckers 83. Thus, the disposable capillary suction tubes 81 and the filter element suckers 83 are separated through the first-time sucker withdrawal, the disposable capillary suction tubes 81 can be separated from the connecting suction tubes 91 through the second-time sucker withdrawal, thus, the filter element suckers 83 can sleeve other suckers to clean the adsorption filter elements 830 in the filter element suckers 83 after sucker withdrawal, and therefore elution of nucleic acid substances is achieved.

In addition, in order to facilitate separation between the disposable capillary suction tubes 81 and the filter element suckers 83, convex rings 831 are arranged on inner circumferential surfaces of upper ends of the filter element suckers 83 in a circumferential direction, and the convex rings 831 abut against outer circumferential surfaces of corresponding positions of the disposable capillary suction tubes 81. Compared with surface-to-surface contact between the outer circumferential surfaces of the disposable capillary suction tubes 81 and inner circumferential surfaces of the filter element suckers 83, friction force of line-to-surface contact between the convex rings 831 and the outer circumferential surfaces of the disposable capillary suction tubes 81 is smaller, and by means of the design, the filter element suckers 83 and the disposable capillary suction tubes 81 can withdraw from the connecting suction tubes 91 correspondingly. First the filter element suckers 83 with small friction force withdraw, then the disposable capillary suction tubes 81 withdraw, and due to the arrangement of the convex rings 831, the disposable capillary suction tubes 81 and the filter element suckers 83 can be sealed. In the embodiment, in order to enable the disposable capillary suction tubes 81 and the filter element suckers 83 to be better sealed, the number of the convex rings 831 arranged up and down is at least two, the two convex rings 831 are arranged at intervals vertically, thus, sealing between the disposable capillary suction tubes and the filter element suckers 83 can further be promoted, and in addition, due to the design of the convex rings 831, the adsorption filter elements 830 can be prevented from disengaging from the filter element suckers 83.

In order to conveniently puncture seals on the reagent strips 7, the nucleic acid extraction device in the invention further comprises a puncture assembly for puncturing the seals on the reagent holes, the puncture assembly comprises puncture frames 141 and puncture heads 14 installed on the puncture frames 141, the puncture heads 14 correspond to the sample holes 31 one to one, and the puncture frames 141 and the piston frames 63 are fixed into a whole. Thus, when the lifting motor 641 drives the liquid transfer table 4 to move downwards; meanwhile, the piston driving apparatuses 61 drive the piston frames 63 to move downwards to drive the pistons 621 to move downwards, the puncture frames 141 are driven to move downwards, and then the puncture heads 14 can move downwards to puncture the seals on the reagent holes of the reagent strips 7. In order to conveniently unload the suckers 82 on the first connectors 621a and the sucker assemblies on the second connectors 911, a sucker withdrawal plate 41 is fixed to the bottom of the liquid transfer table 4, the first sucker withdrawal holes 41a and the second sucker withdrawal holes 41b are formed in the sucker withdrawal plate 41, the first connectors 621a and the second connectors 911 penetrate through the first sucker withdrawal holes 41a and the second sucker withdrawal holes 41b correspondingly, hole diameters of the first sucker withdrawal holes 41a are consistent with radiuses of corresponding positions of the first connectors 621a and smaller than radiuses of connecting ends of the filter element suckers 83, and hole diameters of the second sucker withdrawal holes 41b are consistent with radiuses of corresponding positions of the second connectors 911 and smaller than radiuses of connecting ends of the suckers 82. Guide columns 411 are vertically arranged at two ends of the liquid transfer table 4 correspondingly, and when the piston driving apparatuses 61 drive the piston frames 63 to move to a certain distance downwards, the piston frames 63 can abut against top ends of the guide columns 411. The guide columns 411 penetrate through the liquid transfer table 4, and can move up and down relative to the liquid transfer table 4, lower ends of the guide columns are fixed to the sucker withdrawal plate 41, the guide columns 411 are each of a structure with two ends thicker than a middle, and reset springs 411a penetrate through the middles of the guide columns 411. Specifically, the lower ends of the guide columns 411 penetrate into the liquid transfer table 4, the reset springs 411a are limited between upper ends of the guide columns 411 and the top surface of the liquid transfer table 4. When the piston driving apparatuses 61 drive the piston frames 63 to move to a certain distance downwards, the piston frames 63 abut against top ends of the guide columns 411; thus, the sucker withdrawal plate 41 moves downwards to withdraw the sucker assemblies or the suckers 82, and when pressure acting on the guide columns 411 disappears, the guide columns 411 are reset under the action of the corresponding reset springs 411a. Thus, due to the arrangement of the sucker withdrawal plate 41, the suckers 82 can be unloaded from the first connectors 621a, or the sucker assemblies can be unloaded from the second connectors 911, or the filter element suckers 83 can be unloaded from the disposable capillary suction tubes 81.

The working process of the invention is as follows:
The sample tubes 15 and the reagent strips 7 are put in the sample support 3 and the reagent strip support 231 correspondingly, the nucleic acid extraction device is started, all the motors are reset, and the heating device conducts heating and heat preservation on the samples according to a set heat preservation program. After the samples are subjected to heat preservation, the piston driving apparatuses 61 drive the puncture frames 141 in conjunction with the lifting motor 641, so that the puncture heads 14 puncture the seals on the corresponding reagent holes, to make preparation for subsequent operation.

The sliding motors 221 drive the sliding seat 21, so that the suckers 82 on the sliding seat 21 are right opposite to the first connectors 621a on the liquid transfer table 4 one to one, the lifting motor 641 drives the liquid transfer table 4 to move downwards, and the first connectors 621a press and fetch the suckers 82 and rise. The sliding motor 221 drives the sliding seat 21, so that the first reagent holes 73 containing the diluent are right opposite to the suckers 82 one to one correspondingly, the liquid transfer table 4 falls, the suckers 82 are inserted into the corresponding first reagent holes 73, the piston driving apparatuses 61 work, the suckers 82 suck the diluent, and after the suckers suck the diluent, the lifting motor 641 drives the liquid transfer table 4 to rise.

The sliding seat 21 moves horizontally, so that the sample tubes 15 on the sample support 3 are right opposite to the first connectors 621a on the liquid transfer table 4 one to one, the lifting motor 641 drives the liquid transfer table 4 to move downwards, the piston driving apparatuses 61 work, the diluent in the suckers 82 is added into the corresponding sample tubes 15, the piston parts 62 conduct a plurality of times of suction, to enable the diluent and the samples to be mixed uniformly, and heat preservation is conducted for 5-10min according to requirements.

The lifting motor 641 drives the liquid transfer table 4 to rise, the second sucker holes 72a and 72b are right opposite to the first connectors 621a one to one through the sliding motor 221, the liquid transfer table 4 falls, and the piston driving apparatuses 61 work and drive the sucker withdrawal plate 41 to enable the suckers 82 to withdraw into the corresponding second sucker holes 72a and 72b. The liquid transfer table 4 rises, the sliding seat 21 moves to enable the disposable capillary suction tubes 81 to be right opposite to the second connectors 911 one to one correspondingly, and the liquid transfer table 4 falls, presses the sucker assemblies and rises.

The sliding seat 21 moves to enable the sample tubes 15 to be located below the second connectors 911, the liquid transfer table 4 falls, and the vacuum pumps 5 work to extract the samples. The liquid transfer table 4 rises, the sliding seat 21 moves to enable the first cleaning fluid to be located below the second connectors 911, the liquid transfer table 4 falls, and the vacuum pumps 5 work to extract the first cleaning fluid. The liquid transfer table 4 rises, the sliding seat 21 moves to enable the second cleaning fluid to be located below the second connectors 911, the liquid transfer table 4 falls, and the vacuum pumps 5 work to extract the second cleaning fluid. The liquid transfer table 4 rises, the sliding seat 21 moves to enable the third cleaning fluid to be located below the second connectors 911, the liquid transfer table 4 falls, and the vacuum pumps 5 work to extract the third cleaning fluid. The liquid transfer table 4 rises, the sliding seat 21 moves to enable the fourth cleaning fluid to be located below the second connectors 911, the liquid transfer table 4 falls, the vacuum pumps 5 work to extract the fourth cleaning fluid and meanwhile air-suck and evacuate the filter element suckers 83 for 2-5 min.

The liquid transfer table 4 rises, the sliding seat 21 moves to enable the first sucker holes 71 to be located below the second connectors 911, the liquid transfer table 4 falls, and the piston driving apparatuses 61 work to drive the sucker withdrawal plate 41 to enable the filter element suckers 83 to retreat to the first sucker holes 71. The liquid transfer table 4 rises, the sliding seat 21 moves to enable the first reagent holes 73 to be located below the second connectors 911, the liquid transfer table 4 falls, and the piston driving apparatuses 61 work to drive the sucker withdrawal plate 41 to enable the disposable capillary suction tubes 81 to retreat to the first reagent holes 73 (the diluent in the first reagent holes is evacuated, so that the first reagent holes are used for containing the used disposable capillary suction tubes 81). The liquid transfer table 4 rises, the sliding seat 21 moves to enable the filter element suckers 83 to be located below the first connectors 621a, and the liquid transfer table 4 falls, presses the filter element suckers 83 and rises. The sliding seat 21 moves to enable the third reagent holes 75 to be located below the first connectors 621a, the liquid transfer table 4 falls, and the piston driving apparatuses 61 work to suck the eluant.

After the eluant is sucked, the liquid transfer table 4 rises, the sliding seat 21 moves to enable the fourth reagent holes 76 to be located below the first connectors 621a, the liquid transfer table 4 falls, and the piston driving apparatuses 61 work to push the eluant out.

The liquid transfer table 4 rises, the sliding motor 221 drives the sliding seat 21 to enable the first sucker holes 71 to be located below the first connectors 621a, the liquid transfer table 4 falls, and the piston driving apparatuses 61 work to drive the sucker withdrawal plate 41 to enable the filter element suckers 83 to retreat to the first sucker holes 71 and all the motors are reset.

Compared with the prior art, by means of the nucleic acid extraction device in the invention, automatic and rapid operation of nucleic acid extraction can be achieved and only 5-10min is needed for the whole process. Treatment of a large sample size cannot be achieved within a short time, thus, efficient extraction of nucleic acid is achieved. Since the samples are purified in a suction and cleaning mode, high-purity nucleic acid substances can be obtained, no cross-contamination risk exists, the repeatability is good, and the cost is low.

## Claims

1. A type of nucleic acid extraction device, comprising a workbench (1) with a tabletop (10), **characterized by** further comprising: a sliding seat (21) arranged on the tabletop (10) and capable of being driven by a sliding assembly (22) to slide along the tabletop (10) back and forth; a sample support (3) arranged on the sliding seat (21) and provided with a plurality of sample holes (31) formed side by side in a direction perpendicular to a sliding direction of the sliding seat (21), wherein the sample holes (31) are used for containing sample tubes (15); a reagent strip support (213) arranged on the sliding seat (21), wherein containing grooves (213a) for containing the reagent strips (7) are formed in the reagent strip support, the number of the containing grooves (213a) is consistent with that of the sample holes (3 1), each reagent strip (7) comprises the first sucker hole (71), the second sucker holes (72a and 72 b) and a reagent hole which are formed linearly, the first sucker holes (71) are used for containing sucker assemblies, the second sucker holes (72a and 72b) are used for containing suckers (82), the reagent holes comprise the first reagent holes (73) for containing diluent, the second reagent holes (74a, 74b, 74c and 74d) for containing cleaning fluid, the third reagent holes (75) for containing eluant and the fourth reagent holes (76) for storing target substances, and the first sucker holes (71), the second sucker holes (72a and 72b) and the reagent holes in the reagent strips (7) are opposite to the corresponding sample holes (31) correspondingly when the reagent strips (7) are put in the containing grooves (213a) correspondingly; a liquid transfer table (4) horizontally arranged on a vertical frame (12) fixed to the tabletop (10) and capable of being driven by a lifting assembly (64) to go up and down relative to the sliding seat (21); piston assemblies arranged on the liquid transfer table (4), wherein the piston assemblies comprise piston parts (62) and piston driving apparatuses (61), the number of the piston parts (62) is consistent with that of the sample holes (31), the piston parts (62) comprise piston tubes (622) and pistons (621) arranged in the piston tubes (622) and capable of moving along inner walls of the piston tubes (622) up and down, the piston tubes (622) all vertically penetrate through the liquid transfer table (4), the first connectors (621a) for being in inserted connection with the suckers (82) are formed at lower ends of the piston tubes (622) correspondingly, and the piston driving apparatuses (61) can drive the pistons (621) in the piston parts (62) to move up and down relative to the corresponding piston tubes (622); and suction assemblies comprising connecting suction tubes (91) and vacuum pumps (5), wherein the number of the connecting suction tubes (91) is consistent with that of the sample holes (31), the connecting suction tubes (91) all penetrate through the liquid transfer table (4), upper ends of the connecting suction tubes (91) connect with the vacuum pumps (5) correspondingly, and the second connectors (911) for being in inserted connection with the sucker assemblies are formed at lower ends of the connecting suction tubes correspondingly, wherein the sucker assemblies comprise filter element suckers (83) and disposable capillary suction tubes (81), adsorption filter elements (830) are embedded into the filter element suckers (83), upper ends of the disposable capillary suction tubes (81) sleeve lower ends of the connecting suction tubes (91) and are embedded into upper ports of the filter element suckers (83), and the disposable capillary suction tubes (81) are located in the filter element suckers (83) and located above the absorption filter elements (830).

2. A nucleic acid extraction device according to claim 1, **characterized in that** a heating device for heating the sample support (3) is further arranged on the sliding seat.

3. A nucleic acid extraction device according to claim 1 or 2, **characterized by** further comprising a puncture assembly for puncturing seals on the reagent holes, wherein the puncture assembly comprises puncture frames (141) and puncture heads (14) installed on the puncture frames (141), the puncture heads (14) match the sample holes (31), the pistons (621) are all installed on piston frames (63), the piston frames (63) are fixed to output shafts of the piston driving apparatuses (61), and the puncture frames (141) are fixed to the piston frames (63).

4. A nucleic acid extraction device according to claim 3, **characterized in that** a plurality of vacuum pumps (5) correspond to piston parts (62) one to one, suction connectors (92) are arranged on the piston frames (63), the suction connectors (92) correspond to the piston parts (62) one to one in number, the suction connectors (92) all penetrate through the suction connector frames (65), one ends of suction connectors (92) are connected with connectors on the corresponding vacuum pumps (5) through connecting tubes, and the other ends of the suction connectors are connected with upper-end connectors of the connecting suction tubes (91) through connecting tubes.

5. A nucleic acid extraction device according to claim 4, **characterized in that** an installation frame (51) is arranged on a tabletop (10), and the vacuum pumps (5) are put on the installation frame (51).

6. A nucleic acid extraction device according to claim 1 or 2, **characterized in that** a sliding assembly (22) comprises the first sliding blocks (227), the first sliding rails (226), a pressing block (225), a sliding motor (221), a synchronous belt (224), a driving belt wheel (222) and a driven belt wheel (223); the first sliding blocks (227) are fixed to the bottom of the sliding seat (21), the first sliding rails (226) are fixed to the tabletop (10), the first sliding blocks (227) can slide along the first sliding rails (226) back and forth, the sliding motor (221) is fixed to the tabletop (10), the driving belt wheel (222) is fixed to an output shaft of the sliding motor (221), the driven belt wheel (223) is installed on the tabletop (10), the synchronous belt (224) is tensioned between the driving belt wheel (222) and the driven belt wheel (223), and the pressing block (225) is fixed to one side of the sliding seat (21) and pressed on the synchronous belt (224).

7. A nucleic acid extraction device according to claim I or 2, **characterized in that** the lifting assembly (64) comprises a lifting motor (641), a lifting lead screw (642) and a lifting seat (643), the vertical frame (12) comprises an n-shaped frame (121) and a vertical plate (122) fixed to a top plate of the n-shaped frame (121), the lifting motor (641) is installed at the upper end of the vertical plate (122), the second sliding rail (122b) and a sliding hole (122a) vertically stretch from the position, below the lifting motor (641), of the vertical plate (122), the lifting lead screw (642) is vertically arranged, the upper end of the lifting lead screw is connected with an output shaft of the lifting motor (641), the lower end of the lifting lead screw is connected with the top plate of the n-shaped frame (121), the lifting seat (643) penetrates through the vertical plate (122) through the sliding hole (122a), the front end of the lifting seat is fixed to the liquid transfer table (4), the rear end of the lifting seat sleeves the lead screw (642), a screw transmission pair is formed by the lifting seat and the lead screw (642), the second sliding block (644) is arranged on the lifting seat (643), and when the lifting motor (641) drives the lead screw (642) to rotate, the lead screw (642) can drive the lifting seat (643), and enables the second sliding block (644) to slide along the second sliding rail (122b) up and down, and the corresponding position of the lifting seat (643) can move along the sliding hole (122a) up and down.

8. A nucleic acid extraction device according to claim I or 2, **characterized in that** the reagent strips (7) comprise carrier plates (70), a plurality of the first through holes (701) and the second through holes (702) are formed side by side in the carrier plates (70) in a length direction of the carrier plates, tube bodies (703) corresponding to the first through holes (701) one to one stretch out of one sides of the carrier plates (70); meanwhile, sleeve bodies (704) corresponding to the second through holes (702) one to one further stretch out of one sides of the carrier plates (70), tube openings of the tube bodies (703) are right opposite to the corresponding first through holes (701) correspondingly, openings of the sleeve bodies (704) are right opposite to the corresponding second through holes (701) correspondingly, and therefore the first sucker holes (71), the second sucker holes (72a and 72b) and the reagent holes are formed correspondingly.

9. A nucleic acid extraction device according to claim 8, **characterized in that** the first connecting parts (78) and the second connecting parts (77) are arranged on two sides of the carrier plates (70) correspondingly, and the first connecting parts (78) and the second connecting parts (77) can be detachably connected.

10. A nucleic acid extraction device according to claim 9, **characterized in that** the first connecting parts (78) are connecting columns, and the second connecting parts (77) are connecting sleeves allowing the connecting columns to be inserted therein.

11. A nucleic acid extraction device according to claim 1, **characterized in that** lower ends of the disposable capillary suction tubes (81) are opposite to the adsorption filter elements (830) up and down.

12. A nucleic acid extraction device according to claim 11, **characterized in that** the distance between the lower ends of the disposable capillary suction tubes (81) and the upper surface of the adsorption filter elements (830) up and down is 0-10mm.

13. A nucleic acid extraction device according to claim 12, **characterized in that** the distance between the lower ends of the disposable capillary suction tubes (81) and the upper surface of the adsorption filter elements (830) up and down is 2-5mm.

14. A nucleic acid extraction device according to claim 1, **characterized in that** the disposable capillary suction tubes (81) comprise the first portions (811) located at the upper ends and the second portions (812) located at the lower ends, wherein the first portions (811) are used for being connected to the connecting suction tubes (91) in a sleeving mode, and tube diameters of the second portions (812) enable liquid to smoothly pass through the disposable capillary suction tubes (81) under the action of suction force.

15. A nucleic acid extraction device according to claim 14, **characterized in that** tube diameters of tube bodies of the first portions (811) are 0.5-2.0mm.

16. A nucleic acid extraction device according to claim 1, **characterized in that** edges of upper ports of the disposable capillary suction tubes (81) are lower than those of the upper ports of the filter element suckers (83).

17. A nucleic acid extraction device according to claim 1, **characterized in that** convex rings (831) are arranged on inner circumferential surfaces of upper ends of the filter element suckers (83) in a circumferential direction, and the convex rings (831) abut against outer circumferential surfaces of corresponding positions of the disposable capillary suction tubes (81).

18. A nucleic acid extraction device according to claim 17, **characterized in that** the number of the convex rings (831) arranged vertically is at least two, and the convex rings (831) are arranged vertically at intervals.

## Patentansprüche

1. Vorrichtung zum Extrahieren von Nukleinsäuren, umfassend einen Arbeitstisch (1) mit einer Tischplatte (10), **dadurch gekennzeichnet, dass** sie ferner Folgendes umfasst: einen auf der Tischplatte (10) angeordneten und durch eine Gleitanordnung (22) antreibbaren Gleitstuhl (21), der entlang der Tischplatte (10) hin- und her gleitfähig ist; einen auf dem Gleitstuhl (21) angeordneten Probenhalter (3) mit mehreren nebeneinander in einer zur Gleitbewegung des Gleitstuhls (21) senkrechten Richtung ausgebildeten Probenlöchern (31), wobei die Probenlöcher (31) zur Aufnahme von Probenröhrchen (15) dienen; einen auf dem Gleitstuhl (21) angeordneten Reagenzstreifenhalter (213), wobei in dem Reagenzstreifenhalter Aufnahmenuten (213a) zur Aufnahme der Reagenzstreifen (7) ausgebildet sind, wobei die Anzahl der Aufnahmenuten (213a) der Anzahl der Probenlöcher (31) entspricht, wobei jeder Reagenzstreifen (7) ein erstes Saugerloch (71), zweite Saugerlöcher (72a und 72b) sowie ein Reagenzloch, die linear ausgebildet sind, umfasst, wobei die ersten Saugerlöcher (71) zur Aufnahme von Saugeranordnungen dienen, während die zweiten Saugerlöcher (72a und 72b) zur Aufnahme von Saugern (82) dienen, wobei die Reagenzlöcher erste Reagenzlöcher (73) zur Aufnahme von Verdünnungsmittel, zweite Reagenzlöcher (74a, 74b, 74c und 74d) zur Aufnahme von Reinigungsflüssigkeit, dritte Reagenzlöcher (75) zur Aufnahme von Elutionsmittel und vierte Reagenzlöcher (76) zur Speicherung von Zielsubstanzen umfassen, wobei die ersten Saugerlöcher (71), die zweiten Saugerlöcher (72a und 72b) sowie die Reagenzlöcher der Reagenzstreifen (7) jeweils den entsprechenden Probenlöchern (31) gegenüberliegen, wenn die Reagenzstreifen (7) in die entsprechenden Aufnahmenuten (213a) eingesetzt sind; einen Flüssigkeitstransfertisch (4), der horizontal auf einem an der Tischplatte (10) befestigten Vertikalrahmen (12) angeordnet und durch eine Hubanordnung (64) relativ zum Gleitstuhl (21) auf- und abbewegbar ist; auf dem Flüssigkeitstransfertisch (4) angeordnete Kolbenanordnungen, wobei die Kolbenanordnungen Kolbenteile (62) und Kolbenantriebe (61) umfassen, wobei die Anzahl der Kolbenteile (62) der Anzahl der Probenlöcher (31) entspricht, wobei die Kolbenteile (62) Kolbenröhrchen (622) und in den Kolbenröhrchen (622) angeordnete, entlang der Innenwände der Kolbenröhrchen (622) auf- und abbewegbare Kolben (621) umfassen, wobei die Kolbenröhrchen (622) alle vertikal durch den Flüssigkeitstransfertisch (4) hindurchführen, wobei an den unteren Enden der Kolbenröhrchen (622) jeweils erste Verbinder (62la) zur steckbaren Verbindung mit den Saugern (82) ausgebildet sind und die Kolbenantriebe (61) die Kolben (621) in den Kolbenteilen (62) antreiben können, sich relativ zu den entsprechenden Kolbenröhrchen (622) auf- und abzubewegen; und Sauganordnungen, die Verbindungssaugröhrchen (91) und Vakuumpumpen (5) umfassen, wobei die Anzahl der Verbindungssaugröhrchen (91) der Anzahl der Probenlöcher (31) entspricht, wobei die Verbindungssaugröhrchen (91) alle durch den Flüssigkeitstransfertisch (4) hindurchführen, wobei die oberen Enden der Verbindungssaugröhrchen (91) jeweils mit den Vakuumpumpen (5) verbunden sind und an den unteren Enden der Verbindungssaugröhrchen jeweils zweite Verbinder (911) zur steckbaren Verbindung mit den Saugeranordnungen ausgebildet sind, wobei die Saugeranordnungen Filterelementsauger (83) und Einweg-Kapillarsaugröhrchen (81) umfassen, wobei Adsorptionsfilterelemente (830) in die Filterelementsauger (83) eingesetzt sind, wobei die oberen Enden der Einweg-Kapillarsaugröhrchen (81) die unteren Enden der Verbindungssaugröhrchen (91) umschließen und in die oberen Anschlüsse der Filterelementsauger (83) eingesetzt sind, und wobei die Einweg-Kapillarsaugröhrchen (81) sich in den Filterelementsaugern (83) und oberhalb der Adsorptionsfilterelemente (830) befinden.

2. Vorrichtung zum Extrahieren von Nukleinsäuren nach Anspruch 1, **dadurch gekennzeichnet, dass** auf dem Gleitstuhl ferner eine Heizvorrichtung zum Erhitzen des Probenhalters (3) angeordnet ist.

3. Vorrichtung zum Extrahieren von Nukleinsäuren nach Anspruch 1 oder 2, **dadurch gekennzeichnet, dass** sie ferner eine Durchstoßanordnung zum Durchstoßen von Versiegelungen an den Reagenzlöchern umfasst, wobei die Durchstoßanordnung Durchstoßrahmen (141) und auf den Durchstoßrahmen (141) angebrachte Durchstoßköpfe (14) umfasst, wobei die Durchstoßköpfe (14) den Probenlöchern (31) zugeordnet sind, wobei die Kolben (621) alle an Kolbenrahmen (63) angebracht sind, wobei die Kolbenrahmen (63) an den Abtriebswellen der Kolbenantriebe (61) befestigt sind und die Durchstoßrahmen (141) an den Kolbenrahmen (63) befestigt sind.

4. Vorrichtung zum Extrahieren von Nukleinsäuren nach Anspruch 3, **dadurch gekennzeichnet, dass** mehrere Vakuumpumpen (5) jeweils einem Kolbenteil (62) zugeordnet sind, wobei Saugverbinder (92) an den Kolbenrahmen (63) angeordnet sind, wobei die Anzahl der Saugverbinder (92) der Anzahl der Kolbenteile (62) entspricht, wobei die Saugverbinder (92) alle durch die Saugverbinderrahmen (65) hindurchführen, wobei ein Ende der Saugverbinder (92) über Verbindungsröhrchen mit Anschlüssen der jeweiligen Vakuumpumpe (5) verbunden ist und das andere Ende der Saugverbinder über Verbindungsröhrchen mit dem oberen Ende der Verbindungssaugröhrchen (91) verbunden ist.

5. Vorrichtung zum Extrahieren von Nukleinsäuren nach Anspruch 4, **dadurch gekennzeichnet, dass** auf der Tischplatte (10) ein Montagerahmen (51) angeordnet ist und die Vakuumpumpen (5) auf dem Montagerahmen (51) platziert sind.

6. Vorrichtung zum Extrahieren von Nukleinsäuren nach Anspruch 1 oder 2, **dadurch gekennzeichnet, dass** die Gleitanordnung (22) erste Gleitsteine (227), erste Gleitschienen (226), einen Andruckblock (225), einen Gleitmotor (221), einen Synchronriemen (224), ein Antriebsriemenrad (222) und ein Abtriebsriemenrad (223) umfasst, wobei die ersten Gleitsteine (227) an dem Boden des Gleitstuhls (21) befestigt sind, wobei die ersten Gleitschienen (226) an der Tischplatte (10) befestigt sind, wobei die ersten Gleitsteine (227) entlang der ersten Gleitschienen (226) hin- und hergleiten können, wobei der Gleitmotor (221) an der Tischplatte (10) befestigt ist, wobei das Antriebsriemenrad (222) an einer Abtriebswelle des Gleitmotors (221) befestigt ist, wobei das Abtriebsriemenrad (223) auf der Tischplatte (10) angebracht ist, wobei der Synchronriemen (224) zwischen dem Antriebsriemenrad (222) und dem Abtriebsriemenrad (223) gespannt ist und der Andruckblock (225) an einer Seite des Gleitstuhls (21) befestigt und auf den Synchronriemen (224) gedrückt ist.

7. Vorrichtung zum Extrahieren von Nukleinsäuren nach Anspruch 1 oder 2, **dadurch gekennzeichnet, dass** die Hubanordnung (64) einen Hubmotor (641), eine Hubleitspindel (642) und einen Hubstuhl (643) umfasst, wobei der Vertikalrahmen (12) einen n-förmigen Rahmen (121) und eine an einer Deckplatte des n-förmigen Rahmens (121) befestigte Vertikalplatte (122) umfasst, wobei der Hubmotor (641) am oberen Ende der Vertikalplatte (122) angebracht ist, wobei die zweite Gleitschiene (122b) und ein Gleitloch (122a) sich von der unterhalb des Hubmotors (641) gelegenen Position der Vertikalplatte (122) vertikal erstrecken, wobei die Hubleitspindel (642) vertikal angeordnet ist, wobei das obere Ende der Hubleitspindel mit einer Abtriebswelle des Hubmotors (641) verbunden ist, während das untere Ende der Hubleitspindel mit der Deckplatte des n-förmigen Rahmens (121) verbunden ist, wobei der Hubstuhl (643) durch das Gleitloch (122a) durch die Vertikalplatte (122) hindurchführt, wobei das vordere Ende des Hubstuhls am Flüssigkeitstransfertisch (4) befestigt ist, wobei das hintere Ende des Hubstuhls die Leitspindel (642) umschließt, wobei ein Schraubenübertragungspaar durch den Hubstuhl und die Leitspindel (642) gebildet ist, wobei der zweite Gleitstein (644) am Hubstuhl (643) angeordnet ist, wobei beim Antrieb der Leitspindel (642) zum Drehen durch den Hubmotor (641) die Leitspindel (642) den Hubstuhl (643) antreiben kann und den zweiten Gleitstein (644) entlang der zweiten Gleitschiene (122b) auf- und abgleiten lässt, wobei die entsprechende Position des Hubstuhls (643) entlang des Gleitlochs (122a) auf- und abbewegbar ist.

8. Vorrichtung zum Extrahieren von Nukleinsäuren nach Anspruch 1 oder 2, **dadurch gekennzeichnet, dass** die Reagenzstreifen (7) Trägerplatten (70) umfassen, wobei in den Trägerplatten (70) in Längenrichtung der Trägerplatten mehrere erste Durchgangslöcher (701) und zweite Durchgangslöcher (702) nebeneinander ausgebildet sind, wobei Rohrkörper (703), die den ersten Durchgangslöchern (701) jeweils zugeordnet sind, an einer Seite der Trägerplatten (70) herausragen, wobei Hüllkörper (704), die den zweiten Durchgangslöchern (702) jeweils zugeordnet sind, an einer Seite der Trägerplatten (70) weiterhin herausragen, wobei Rohröffnungen der Rohrkörper (703) den entsprechenden ersten Durchgangslöchern (701) jeweils gegenüberliegen, wobei Öffnungen der Hüllkörper (704) den entsprechenden zweiten Durchgangslöchern (702) jeweils gegenüberliegen und dadurch die ersten Saugerlöcher (71), die zweiten Saugerlöcher (72a und 72b) sowie die Reagenzlöcher entsprechend gebildet sind.

9. Vorrichtung zum Extrahieren von Nukleinsäuren nach Anspruch 8, **dadurch gekennzeichnet, dass** an den beiden Seiten der Trägerplatten (70) die ersten Verbindungsteile (78) und die zweiten Verbindungsteile (77) jeweils angeordnet sind und die ersten Verbindungsteile (78) und die zweiten Verbindungsteile (77) lösbar miteinander verbindbar sind.

10. Vorrichtung zum Extrahieren von Nukleinsäuren nach Anspruch 9, **dadurch gekennzeichnet, dass** die ersten Verbindungsteile (78) Verbindungssäulen sind und die zweiten Verbindungsteile (77) Verbindungshülsen sind, in welche die Verbindungssäulen eingesteckt werden dürfen.

11. Vorrichtung zum Extrahieren von Nukleinsäuren nach Anspruch 1, **dadurch gekennzeichnet, dass** die unteren Enden der Einweg-Kapillarsaugröhrchen (81) den Adsorptionsfilterelementen (830) in vertikaler Richtung gegenüberliegen.

12. Vorrichtung zum Extrahieren von Nukleinsäuren nach Anspruch 11, **dadurch gekennzeichnet, dass** ein Abstand zwischen den unteren Enden der Einweg-Kapillarsaugröhrchen (81) und der Oberfläche der Adsorptionsfilterelemente (830) in vertikaler Richtung 0 bis 10 mm beträgt.

13. Vorrichtung zum Extrahieren von Nukleinsäuren nach Anspruch 12, **dadurch gekennzeichnet, dass** ein Abstand zwischen den unteren Enden der Einweg-Kapillarsaugröhrchen (81) und der Oberfläche der Adsorptionsfilterelemente (830) in vertikaler Richtung 2 bis 5 mm beträgt.

14. Vorrichtung zum Extrahieren von Nukleinsäuren nach Anspruch 1, **dadurch gekennzeichnet, dass** die Einweg-Kapillarsaugröhrchen (81) erste Abschnitte (811) an den oberen Enden und zweite Abschnitte (812) an den unteren Enden umfassen, wobei die ersten Abschnitte (811) in Hülsenmodus mit den Verbindungssaugröhrchen (91) dienen und die Rohrdurchmesser der zweiten Abschnitte (812) so bemessen sind, dass Flüssigkeit unter Einwirkung von Saugkraft problemlos durch die Einweg-Kapillarsaugröhrchen (81) strömen kann.

15. Vorrichtung zum Extrahieren von Nukleinsäuren nach Anspruch 14, **dadurch gekennzeichnet, dass** die Rohrdurchmesser der Rohrkörper der ersten Abschnitte (811) 0,5 bis 2,0 mm betragen.

16. Vorrichtung zum Extrahieren von Nukleinsäuren nach Anspruch 1, **dadurch gekennzeichnet, dass** die Ränder der oberen Anschlüsse der Einweg-Kapillarsaugröhrchen (81) niedriger als die der oberen Anschlüsse der Filterelementsauger (83) liegen.

17. Vorrichtung zum Extrahieren von Nukleinsäuren nach Anspruch 1, **dadurch gekennzeichnet, dass** an den Innenumfangsflächen der oberen Enden der Filterelementsauger (83) in Umfangsrichtung Vorsprungringe (831) angeordnet sind, wobei die Vorsprungringe (831) an den Außenumfangsflächen der entsprechenden Positionen der Einweg-Kapillarsaugröhrchen (81) anliegen.

18. Vorrichtung zum Extrahieren von Nukleinsäuren nach Anspruch 17, **dadurch gekennzeichnet, dass** die Anzahl der vertikal angeordneten Vorsprungringe (831) mindestens zwei beträgt und die Vorsprungringe (831) in vertikalen Abständen angeordnet sind.

## Revendications

1. Un type de dispositif d'extraction d'acides nucléiques, comprenant une table de travail (1) pourvue d'un dessus de table (10), **caractérisé en ce qu'**il comprend en outre : un siège coulissant (21) disposé sur le dessus de table (10) et pouvant être entraîné par un ensemble coulissant (22) pour coulisser d'avant en arrière sur le dessus de table (10) ; un support d'échantillon (3) disposé sur le siège coulissant (21) et pourvu d'une pluralité de trous d'échantillon (31) formés côte à côte dans une direction perpendiculaire à une direction de coulissement du siège coulissant (21), dans lequel les trous d'échantillon (31) sont utilisés pour recevoir des tubes d'échantillon (15) ; un support de bandelette réactive (213) disposé sur le siège coulissant (21), dans lequel des rainures de réception (213a) destinées à recevoir les bandelettes réactives (7) sont formées dans le support de bandelette réactive, le nombre de rainures de réception (213a) est identique à celui de trous d'échantillon (31), chaque bandelette réactive (7) comprend un premier trou de ventouse (71), des seconds trous de ventouse (72a et 72b) et un trou de réactif qui sont formés linéairement, les premiers trous de ventouse (71) sont utilisés pour recevoir des ensemble ventouses, les seconds trous de ventouse (72a et 72b) sont utilisés pour recevoir des ventouses (82), les trous de réactif comprend les premiers trous de réactif (73) destinés à recevoir un diluant, les deuxièmes trous de réactif (74a, 74b, 74c et 74d) destinés à recevoir un fluide de nettoyage, les troisièmes trous de réactif (75) destinés à recevoir un éluant et les quatrièmes trous de réactif (76) destinés à stocker des substances cibles, et les premiers trous de ventouse (71), les seconds trous de ventouse (72a et 72b) et les trous de réactif dans les bandelettes réactives (7) sont respectivement opposés aux trous d'échantillon (31) correspondants lorsque les bandelettes réactives (7) sont respectivement mises dans les rainures de réception (213a) ; une table de transfert de liquide (4) disposée horizontalement sur un cadre vertical (12) fixé au dessus de table (10) et pouvant être entraînée par un ensemble de levage (64) pour monter et descendre par rapport au siège coulissant (21) ; des ensembles pistons disposés sur la table de transfert de liquide (4), dans lequel les ensembles pistons comprennent des parties de piston (62) et des appareils d'entraînement de piston (61), le nombre de parties de piston (62) est identique à celui de trous d'échantillon (31), les parties de piston (62) comprennent des tubes de piston (622) et des pistons (621) disposés dans les tubes de piston (622) et pouvant se déplacer de haut en bas le long de parois internes des tubes de piston (622), les tubes de piston (622) pénètrent tous verticalement à travers la table de transfert de liquide (4), des premiers éléments de raccordement (621a) permettant un raccordement par insertion avec les ventouses (82) sont formés au niveau d'extrémités inférieures des tubes de piston (622), respectivement, et les appareils d'entraînement de piston (61) peuvent entraîner les pistons (621) dans les parties de piston (62) pour qu'ils se déplacent de haut en bas par rapport aux tubes de piston (622) correspondants ; et des ensembles d'aspiration comprenant des tubes d'aspiration de raccordement (91) et des pompes à vide (5), dans lequel le nombre de tubes d'aspiration de raccordement (91) est identique à celui des trous d'échantillon (31), les tubes d'aspiration de raccordement (91) pénètrent tous à travers la table de transfert de liquide (4), des extrémités supérieures des tubes d'aspiration de raccordement (91) sont respectivement raccordées aux pompes à vide (5), et les seconds éléments de raccordement (911) permettant un raccordement par insertion avec les ensemble ventouses sont formés au niveau d'extrémités inférieures des tubes d'aspiration de raccordement, respectivement, dans lequel les ensembles ventouses comprennent des ventouses d'élément filtrant (83) et des tubes d'aspiration capillaires jetables (81), des éléments filtrants d'adsorption (830) sont intégrés dans les ventouses d'élément filtrant (83), des extrémités supérieures des tubes d'aspiration capillaires jetables (81) manchonnent sur des extrémités inférieures des tubes d'aspiration de raccordement (91) et sont intégrées dans des orifices supérieurs des ventouses d'élément filtrant (83), et les tubes d'aspiration capillaires jetables (81) sont situés dans les ventouses d'élément filtrant (83) et situés au-dessus des éléments filtrants d'adsorption (830).

2. Dispositif d'extraction d'acides nucléiques selon la revendication 1, **caractérisé en ce qu'**un dispositif de chauffage destiné à chauffer le support d'échantillon (3) est en outre disposé sur le siège coulissant.

3. Dispositif d'extraction d'acides nucléiques selon la revendication 1 ou 2, **caractérisé en ce qu'**il comprend en outre un ensemble de perforation destiné à perforer des éléments d'étanchéité sur les trous de réactif, dans lequel l'ensemble de perforation comprend des cadres de perforation (141) et des têtes de perforation (14) installées sur les cadres de perforation (141), les têtes de perforation (14) correspondent aux trous d'échantillon (31), les pistons (621) sont tous installés sur des cadres de piston (63), les cadres de piston (63) sont fixés à des arbres de sortie des appareils d'entraînement de piston (61), et les cadres de perforation (141) sont fixés aux cadres de piston (63).

4. Dispositif d'extraction d'acides nucléiques selon la revendication 3, **caractérisé en ce que**, une pluralité de pompes à vide (5) correspondent une à une à des parties de piston (62), des éléments de raccordement d'aspiration (92) sont disposés sur les cadres de piston (63), les éléments de raccordement d'aspiration (92) correspondent un à un aux parties de piston (62) en nombre, les éléments de raccordement d'aspiration (92) pénètrent tous à travers les cadres de élément de raccordement d'aspiration (65), une extrémité d'éléments de raccordement d'aspiration (92) est raccordée à des éléments de raccordement sur les pompes à vide (5) correspondantes par des tubes de raccordement, et l'autre extrémité des éléments de raccordement d'aspiration est raccordée à des éléments de raccordement d'extrémité supérieure des tubes d'aspiration de raccordement (91) par des tubes de raccordement.

5. Dispositif d'extraction d'acides nucléiques selon la revendication 4, **caractérisé en ce qu'**un cadre d'installation (51) est disposé sur un dessus de table (10), et les pompes à vide (5) sont mises sur le cadre d'installation (51).

6. Dispositif d'extraction d'acides nucléiques selon la revendication 1 ou 2, **caractérisé en ce que**, l'ensemble coulissant (22) comprend des premiers blocs coulissants (227), des premiers rails coulissants (226), un bloc de pression (225), un moteur coulissement (221), une courroie synchrone (224), une poulie de courroie d'entraînement (222) et une poulie de courroie entraînée (223) ; les premiers blocs coulissants (227) sont fixés à la partie inférieure du siège coulissant (21), les premiers rails coulissants (226) sont fixés au dessus de table (10), les premiers blocs coulissants (227) peuvent coulisser d'avant en arrière le long des premiers rails coulissants (226), le moteur coulissement (221) est fixé au dessus de table (10), la poulie de courroie d'entraînement (222) est fixée à un arbre de sortie du moteur coulissement (221), la poulie de courroie entraînée (223) est installée sur le dessus de table (10), la courroie synchrone (224) est tendue entre la poulie de courroie d'entraînement (222) et la poulie de courroie entraînée (223), et le bloc de pression (225) est fixé sur un côté du siège coulissant (21) et s'appuie sur la courroie synchrone (224).

7. Dispositif d'extraction d'acides nucléiques selon la revendication 1 ou 2, **caractérisé en ce que**, l'ensemble de levage (64) comprend un moteur de levage (641), une vis de commande (642) de levage et un siège de levage (643), le cadre vertical (12) comprend un cadre en forme de n (121) et une plaque verticale (122) fixée à une plaque supérieure du cadre en forme de n (121), le moteur de levage (641) est installé au niveau de l'extrémité supérieure de la plaque verticale (122), le second rail coulissant (122b) et un trou coulissant (122a) s'étendent verticalement à partir de la position, située au-dessous du moteur de levage (641), de la plaque verticale (122), la vis de commande (642) de levage est disposée verticalement, l'extrémité supérieure de la vis de commande de levage est raccordée à un arbre de sortie du moteur de levage (641), l'extrémité inférieure de la vis de commande de levage est raccordée à la plaque supérieure du cadre en forme de n (121), le siège de levage (643) pénètre à travers la plaque verticale (122) via le trou coulissant (122a), l'extrémité avant du siège de levage est fixée à la table de transfert de liquide (4), l'extrémité arrière du siège de levage manchonne sur la vis de commande (642), une paire de transmission à vis est formé par le siège de levage et la vis de commande (642), le second bloc coulissant (644) est disposé sur le siège de levage (643), et lorsque le moteur de levage (641) entraîne une rotation de la vis de commande (642), la vis de commande (642) peut entraîner le siège de levage (643), et permet au second bloc coulissant (644) de coulisser de haut en bas le long du second rail coulissant (122b), et la position correspondante du siège de levage (643) peut se déplacer de haut en bas le long du trou coulissant (122a).

8. Dispositif d'extraction d'acides nucléiques selon la revendication 1 ou 2, **caractérisé en ce que**, les bandelettes réactives (7) comprennent des plaques porteuses (70), une pluralité de premiers trous traversants (701) et de seconds trous traversants (702) sont formés côte à côte dans les plaques porteuses (70) dans une direction de la longueur des plaques porteuses, des corps de tube (703) qui correspondent un à un aux premiers trous traversants (701) s'étendent de côtés des plaques porteuses (70) ; cependant, des corps de manchon (704) qui correspondent un à un aux seconds trous traversants (702) s'étendent en outre de côtés des plaques porteuses (70), des ouvertures de tube des corps de tube (703) se trouvent juste en face des premiers trous traversants (701) correspondants, des ouvertures des corps de manchon (704) se trouvent juste en face des seconds trous traversants (701), respectivement, et ainsi, les premiers trous de ventouse (71), les seconds trous de ventouse (72a et 72b) et les trous de réactif sont formés en conséquence.

9. Dispositif d'extraction d'acides nucléiques selon la revendication 8, **caractérisé en ce que**, les premières parties de raccordement (78) et les secondes parties de raccordement (77) sont disposées respectivement sur deux côtés des plaques porteuses (70), et les premières parties de raccordement (78) et les secondes parties de raccordement (77) peuvent être raccordées de manière détachable.

10. Dispositif d'extraction d'acides nucléiques selon la revendication 9, **caractérisé en ce que**, les premières parties de raccordement (78) sont des colonnes de raccordement, et les secondes parties de raccordement (77) sont des manchons de raccordement permettant aux colonnes de raccordement d'être insérées dans ceux-ci.

11. Dispositif d'extraction d'acides nucléiques selon la revendication 1, **caractérisé en ce que** des extrémités inférieures des tubes d'aspiration capillaires jetables (81) sont opposées aux éléments filtrants d'adsorption (830), de haut en bas.

12. Dispositif d'extraction d'acides nucléiques selon la revendication 11, **caractérisé en ce que** la distance entre les extrémités inférieures des tubes d'aspiration capillaires jetables (81) et la surface supérieure des éléments filtrants d'adsorption (830), de haut en bas, est comprise entre 0 et 10 mm.

13. Dispositif d'extraction d'acides nucléiques selon la revendication 12, **caractérisé en ce que** la distance entre les extrémités inférieures des tubes d'aspiration capillaires jetables (81) et la surface supérieure des éléments filtrants d'adsorption (830), de haut en bas, est comprise entre 2 et 5 mm.

14. Dispositif d'extraction d'acides nucléiques selon la revendication 1, **caractérisé en ce que**, les tubes d'aspiration capillaires jetables (81) comprennent les premières parties (811) situées au niveau des extrémités supérieures et des secondes parties (812) situées au niveau des extrémités inférieures, dans lequel les premières parties (811) sont utilisées pour être raccordées aux tubes d'aspiration de raccordement (91) dans un mode de manchon, et des diamètres de tube des secondes parties (812) permettent à un liquide de s'écouler sans encombre à travers les tubes d'aspiration capillaires jetables (81) sous l'effet de l'une force d'aspiration.

15. Dispositif d'extraction d'acides nucléiques selon la revendication 14, **caractérisé en ce que** des diamètres de tube de corps de tube des premières parties (811) sont compris entre 0,5 et 2,0 mm.

16. Dispositif d'extraction d'acides nucléiques selon la revendication 1, **caractérisé en ce que** des bords d'orifices supérieurs des tubes d'aspiration capillaires jetables (81) sont plus bas que ceux des orifices supérieurs des ventouses d'élément filtrant (83).

17. Dispositif d'extraction d'acides nucléiques selon la revendication 1, **caractérisé en ce que**, des anneaux convexes (831) sont disposés sur des surfaces circonférentielles internes d'extrémités supérieures des ventouses d'élément filtrant (83) dans une direction circonférentielle, et les anneaux convexes (831) viennent en butée contre des surfaces circonférentielles externes de positions correspondantes des tubes d'aspiration capillaires jetables (81).

18. Dispositif d'extraction d'acides nucléiques selon la revendication 17, **caractérisé en ce que** le nombre d'anneaux convexes (831) disposés verticalement est d'au moins deux, et les anneaux convexes (831) sont disposés verticalement à intervalles.
